# EUROPEAN PATENT APPLICATION

(11) **EP 4 527 342 A1**
(43) Date of publication of application: **26.03.2025**
(21) Application number: 23198964.1
(22) Date of filing: 22.09.2023
(51) Int. Cl.: A61B 90/70

(54) **BASIN FOR CLEANING A TOOL USED DURING A HISTOLOGY PROCEDURE SUCH AS A GROSSING PROCEDURE**

(71) Applicant: Milestone S.r.l., 24010 Sorisole (BG) (IT)
(72) Inventor: Visinoni, Francesco, Mozzo (BG) (IT); Minuti, Matteo, 24053 Brignano Gera d Adda (BG) (IT); Roncalli, Omar, 24040 Bonate Sopra (BG) (IT)
(74) Representative: Kiwit, Benedikt

(57) **Abstract**

A basin (1) for cleaning a tool used during a histology procedure such as a grossing procedure. The basin (1) comprises: a cleaning chamber (10) delimited by a side wall (11) extending from a bottom (12); an inlet conduit (30) arranged to deliver a cleaning liquid via an outlet opening (31) into the cleaning chamber (10), wherein the inlet conduit (30) extends through the side wall (11) and/or the bottom (12); and a liquid conditioning conduit (50) arranged to subject the cleaning liquid to a venturi effect and/or to cavitation upstream of the outlet opening (31).

## Description

### TECHNICAL FIELD OF THE INVENTION

The invention relates to a basin for cleaning a tool used during a histology procedure (such as a grossing procedure), a method for cleaning a tool used during a histology procedure, and a use of such a basin in a histological pathway.

### BACKGROUND OF THE INVENTION

For a histological examination of a human or animal tissue specimen, the specimen is excided from the patient by a clinician in an ambulatory environment or by a surgeon during a surgery and then placed in a container. Standard practices call for using containers of different shapes and volume (e.g., buckets, bags, vials) filled with a fixative, wherein in most cases the fixative is neutral buffered formalin 10%, in which the specimen is placed. Then, the container with the specimen is sent to the histology laboratory or anatomical pathology laboratory for proper analysis. After the histology laboratory has received the tissue specimen, the first operations performed by an operator are the accessioning and grossing.

The accessioning consists of a registration of the incoming tissue specimen and insertion of the data in the Laboratory Information System (LIS). In this phase, a unique tissue code is assigned to the tissue specimen.

During the following grossing step, the tissue specimen is removed from the carrier, e.g., a transportation carrier, and reduced and sampled in a way that is suitable for the next processes. Although reduction and sampling are different with large specimens and minute biopsy specimens, the main goal remains the same in ensuring that as many diagnostic features as possible are visible on the microscope slide.

The reduced tissue specimen is subsequently placed inside a tissue carrier. The reduced tissue specimen must be small enough to be contained inside the tissue carrier, which is, e.g., a standard (histological) tissue cassette. Some tissue specimens, e.g., small biopsies, are usually already small enough so that the reduction is not required.

The tools used during the grossing for performing the reduction, sampling and tissue transfer into the tissue carrier are mainly forceps, tweezers, knives and scissors. Such tools can be reused for many tissue specimens. The cleaning of the tools between the grossing of different tissue specimens is crucial. In fact, after the grossing of a tissue specimen some tissue fragments may remain stuck on the tool (e.g., forceps, a tweezer, or a knife) and, if not removed, these fragments may lead to a carry-over and cross-contamination of the following tissue specimens, that may lead to a wrong diagnosis.

According to a study regarding contamination of tissue specimens along the entire process of manipulation, over 90% of extraneous tissue was thought to originate from the pathology laboratory with an overall extraneous tissue rate of 2.9% on retrospectively reviewed slides (Richard J Zarbo; The Unsafe Archaic Processes of Tissue Pathology: Manifesto for Change; American Journal of Clinical Pathology, Volume 158, Issue 1, July 2022, Pages 4-7); (Gephardt GN, Zarbo RJ. Extraneous tissue in surgical pathology: a College of American Pathologists Q-Probes study of 275 laboratories. Arch Pathol Lab Med. 1996;120:1009-1014). Extraneous tissue may pose a severe diagnostic dilemma increasing the risk of a wrong diagnosis. An important part of such extraneous tissues is related to incomplete or not effective cleaning of the grossing tools.

The simple rinse under the tap water is not enough to effectively remove the fragments (that, for example, may remain in the serrated part of the tweezer tips), while using brushes or sponges or the like may lead to a collection of fragments on the surface of said brushes or sponges that can be passed back to tweezers or the like during the following cleaning.

Other methods, such as sterilization, gamma radiation, ultrasound, autoclave or the like, may be very effective but too slow and discontinuous for the daily routine, during which only few seconds for tools cleaning are expected.

Furthermore, a hypothetical solution involving the use of disposable single-use tools is economically disadvantageous as well as environmentally unfriendly.

Thus, it is an objective to overcome one or more of the previous drawbacks.

### SUMMARY OF THE INVENTION

The object of the present invention is achieved by the solution provided in the enclosed independent claims. Advantageous implementations of the present invention are further defined in the dependent claims.

According to a first aspect, the invention relates to a basin for cleaning a tool used during a histology procedure such as a grossing procedure. The basin comprises: a cleaning chamber delimited by a side wall extending from a bottom; an inlet conduit arranged to deliver a cleaning liquid via an outlet opening into the cleaning chamber, wherein the inlet conduit extends through the side wall and/or the bottom; and a liquid conditioning conduit arranged to subject the cleaning liquid to a venturi effect and/or to cavitation upstream of the outlet opening.

The basin provides the advantage of effectively cleaning a tool used during a histology procedure such as a grossing procedure. By the basin, the cleaning liquid within the cleaning chamber moves not only due to being delivered, by the inlet conduit, into the cleaning chamber, but also due to traversing the liquid conditioning conduit. The liquid conditioning conduit in particular can transfer the cleaning liquid in such a condition that the tool can undergo a cleaning (rinsing, washing, etc.) within the cleaning chamber in a particularly effective manner. By the venturi effect and/or the cavitation effected by at least the liquid conditioning conduit, the cleaning liquid is, in particular, in an agitated condition within the cleaning chamber, thereby effectively cleaning the tool therein. Further, by the liquid conditioning conduit, the basin can provide an effective cleaning without requiring much space. For example, complex cleaning means for sterilisation, gamma radiation and/or ultra sound, such as an autoclave, can be omitted, while still providing an effective cleaning of the tool.

The basin may be used in different stages of tissue sample manipulation, e.g., at least during or after the grossing. The basin may be used as an add-on module for a (stationary) basin. For example, the basin comprises a support structure (e.g. a chassis) supporting (directly or indirectly) the components (i.e., at least the cleaning chamber, the inlet conduit and the liquid conditioning conduit).

The liquid conditioning conduit may comprise, or may be, a venturi tube. This provides the advantage of achieving the venturi effect and/or the cavitation in a particularly easy manner.

The liquid conditioning conduit may comprise: an inlet opening connectable to a liquid source (such as tap water or mains water), an outlet opening connected to the inlet conduit, and a constricted section arranged between the inlet opening and the outlet opening of the liquid conditioning conduit.

By the constricted section, a pressure drop of the cleaning liquid within the liquid conditioning conduit can be easily achieved. The pressure drop may be such that a gas can be drawn, or sucked, into the cleaning liquid being in the constricted section. For example, the pressure drop effects a pressure within the constricted section, wherein this pressure is below an outside (atmospheric) pressure, thereby effecting a flow of the gas into the cleaning liquid being in the constricted section. By the gas enclosed in the cleaning liquid, the cleaning liquid moves in the cleaning chamber in a manner that is particularly effective for cleaning the tool. Additionally, or alternatively, the pressure drop may be such that the (static) pressure of the cleaning liquid within the constricted section is reduced to below the liquid's vapour pressure, leading to cavitation, i.e., the formation of small vapour-filled cavities in the cleaning liquid. By these cavities, the cleaning liquid moves in the cleaning chamber in a manner that is particularly effective for cleaning the tool.

The venturi effect and/or the cavitation can thus effect that gas (such as air) bubbles and/or small vapour-filled cavities are present in the cleaning liquid delivered via the outlet opening into the cleaning chamber. Thereby, the cleaning liquid within the cleaning chamber is particularly well agitated and/or in a condition of turbulence, whereby the tool within the cleaning chamber can undergo a particularly effective cleaning (rinsing, washing, etc.).

The constricted section may comprise a suction opening (e.g., a hole such as a drilled hole) through which a gas, such as air, can be drawn from an area outside of the liquid conditioning conduit and into a (cleaning) liquid within the constricted section. This achieves the advantage that the cleaning liquid can be transferred into a condition advantageous for cleaning in a particularly effective manner. The reduced pressure within the constricted section may effect a flow from the area via the suction opening into the (constricted) channel (and thus the therein provided liquid) of the constricted section.

Thus, the liquid conditioning conduit may have three fluid connections: one first inlet connection for the liquid flow source, one second inlet connection for the gas (e.g., air) source and one outlet connection to be connected, via the inlet conduit, to the cleaning chamber. The first inlet and the outlet may be reciprocally coaxial, while the second inlet may be perpendicular or almost perpendicular in respect to the first inlet and outlet. The liquid conditioning conduit may comprise a fluid path of varying diameter between the first inlet and the outlet, and the second inlet is connected to said fluid path in the throat, where (or almost where) the diameter is smaller.

The constricted section may be void of any suction opening through which a gas, such as air, can be drawn from an area outside of the liquid conditioning conduit and into a liquid within the constricted section. This achieves the advantage that the liquid conditioning conduit can be provided in an easy manner, while still having the possibility to condition the cleaning liquid within the constricted section, such as by at least cavitation. For example, the liquid conditioning conduit may comprise only a single channel (having different sections), wherein this channel may extend from the inlet opening to the outlet opening of the liquid conditioning conduit.

The liquid conditioning conduit is preferably made of plastic or metal material.

The side wall may have a curved cross section. For example, the side wall may form, or may be part of, a curved (e.g., cylindrical) body.

The inlet conduit and/or the liquid conditioning conduit preferably do/does not extend radially with respect to the side wall and/or the (at least partly curved) cleaning chamber. Thereby, the cleaning liquid may be delivered into the cleaning chamber in a way that allows for a particularly effective movement of the cleaning liquid within the cleaning chamber. Accordingly, the tool within the cleaning chamber can be cleaned in a particularly effective manner. Additionally, or alternatively, the inlet conduit and/or the liquid conditioning conduit may extend/s tangentially with respect to the side wall.

The basin may comprise a lid. The lid in particular provides the advantage that the cleaning liquid moving within the cleaning chamber does not spill and/or splash out of the cleaning chamber.

The lid is preferably arranged to cover at least part of an opening defined by the side wall or by a flange extending from the side wall. Preferably, the lid is arranged to cover only a part of the opening. As such, the lid can, in a closed state of the cleaning chamber, still provide some access to the cleaning chamber, e.g. for cleaning a tool.

The lid may be attached to the side wall and/or the flange, e.g. by a fastening means such as one or more screws.

The lid may comprise a tool opening arranged to receive a tool being cleaned in the cleaning chamber. Thus, a user can clean, by the basin, a tool without being much exposed to the cleaning liquid moving within the cleaning chamber. Preferably, the lid can, in a closed state of the cleaning chamber, provide access to the cleaning chamber exclusively via the tool opening. The tool opening may have the form of a slot. The tool opening may be arranged so as to help, on a side of the top of the lid, to place one or more tools right within the flow discharged via the outlet opening of the inlet conduit. For example, the tool opening facilitates that a tool received in the tool opening can be placed in front of the liquid conditioning conduit. The tool opening may comprise a plurality of tool openings (e.g. with identical or different shapes) which may be arranged such that tools received by these tool openings are arranged in a defined position in relation to the outlet opening of the inlet conduit or the liquid flow discharged therefrom.

The lid may comprise a mechanical positioning means adapted to interact with a part of the basin in such a way that the tool opening is in a defined position with respect to the side wall and/or the outlet opening of the inlet conduit. This has the advantage that a tool received by the tool opening can be arranged in a specific area within the cleaning chamber, where the moving cleaning liquid is particularly effective for cleaning the tool.

The basin may comprise a drain opening for draining liquid from the cleaning chamber. As such, it can be ensured that liquid within the cleaning chamber does not exceed a specific level. The drain opening may comprise a plurality of drain openings.

The bottom may comprise the drain opening. Additionally, or alternatively, the side wall may comprise the drain opening. Arranged in the side wall, the drain opening may form an overflow opening/protection.

The drain opening may comprise one or more first drain openings and one or more second drain openings, wherein the bottom comprises the one or more first drain openings and the side wall comprises the one or more second drain openings.

The drain opening may comprise a filter. In particular, the filter may be designed to withhold tissue fragments removed from the tool during cleaning inside the cleaning chamber. The filter can be useful to avoid an unnoticed or unintentional loss of useful tissue specimen that may have been wrongly left on the tool before cleaning.

The filter may be removable, e.g. for being cleaned and avoid a drain clogging.

At least part or the bottom may be inclined or oblique, e.g. with respect to the side wall and/or the lid. The inclination in particular achieves the advantage that the cleaning chamber can be completely emptied in an easy manner. Additionally, or alternatively, at least part of the bottom may be flat or horizontal, such as perpendicular with respect to the side wall.

Preferably, at least part of the bottom may be inclined (e.g., with respect to the side wall and/or the lid) in such a way that liquid on the bottom can flow by gravity towards the drain opening.

At least part (and preferably the entirety) of the liquid conditioning conduit may be arranged outside of the cleaning chamber.

At least part of the inlet conduit may be arranged inside of, and/or extend (protrude, etc.) into, the cleaning chamber.

The basin may further comprise: a valve arranged upstream of the liquid conditioning conduit, wherein the valve is preferably at least a time-controlled valve; and/or a pressure and/or flow regulator arranged to control a pressure and/or flow of a liquid flowing towards, or in, the inlet conduit; and/or a heater arranged to heat, preferably upstream of the cleaning chamber, the cleaning liquid or an ingredient (such as tap water and/or another ingredient such as a detergent) of the cleaning liquid.

Providing the heater may have an advantageous effect for conditioning the liquid by cavitation. In particular, by the heater the temperature of the liquid can be increased such that the so heated liquid can be more easily subjected to cavitation in the downstream liquid conditioning conduit.

The valve may be operated manually through a lever/pushbutton or with an automatic detection sensor. The valve may be time-controlled in such a way that it is in an open position only for a period of less than 1 minute, such as for period in a range of 5-30 seconds, such as of 5-15 seconds, e.g. 10 seconds.

The pressure and/or flow regulator and/or the heater may be connected to an inlet of the valve, wherein an outlet of the valve may be connected to the liquid conditioning conduit. By the pressure and/or flow regulator, a flow adjustment of the liquid can be achieved, thereby reducing the cleaning time and/or improving the effectiveness of cleaning. Similarly, by the heater, heating of the liquid can be achieved, thereby reducing the cleaning time and/or improving the effectiveness of cleaning.

The basin may comprise a control element for controlling a liquid stream, such as a stream of the cleaning liquid delivered into the cleaning chamber. The user control element may comprise a touch-sensitive (such as capacitive and/or resistive) control element and/or a mechanical control element (such as a mechanical button).

The basin may comprise a plate that receives the cleaning chamber and the user control element. For example, the side wall and/or the lid is/are attached to the plate. The plate may extend along a direction perpendicular to the side wall and/or parallel to the lid.

The user control element may be for controlling the valve and/or the pressure and/or flow regulator, and/or the heater.

For example, a user may operate the user control element in order to open the valve. In the open state, the valve allows liquid (such as tap water) to flow within the liquid conditioning conduit and, after this, into the cleaning chamber. After a defined period of time, such as after a time for properly cleaning the tool, the valve may, due to its configuration of being time-controlled, switch into the closed state, thereby stopping a flow of liquid from the liquid source into the liquid conditioning conduit.

The supporting structure (e.g. a chassis) is preferably made of stainless steel or similar material. As an option, the supporting structure may also support, and/or accommodate. A tap for generic use may also be received by the supporting structure.

According to a second aspect, the invention relates to a method for cleaning a tool used during a histology procedure such as a grossing procedure, wherein the method comprises the steps of: a) providing a basin as described above; b) subjecting a cleaning liquid, by the liquid conditioning conduit, to a venturi effect and/or cavitation upstream of the outlet opening; c) delivering, by the inlet conduit and the outlet opening, the conditioned cleaning liquid, i.e. the cleaning liquid conditioned by step b), into the cleaning chamber; d) cleaning, by the delivered cleaning liquid, i.e. by the cleaning liquid delivered by step c), the tool in the cleaning chamber; and e) optionally reusing the cleaned tool in a histology procedure such as a grossing procedure.

According to a third aspect, the invention relates to a use of the basin as described above in a histological pathway, such as during or after grossing.

### BRIEF DESCRIPTION OF THE DRAWINGS

The invention will be explained in the following together with the figures.
- Fig. 1: shows a schematic perspective view of a basin according to an embodiment;
- Fig. 2: shows a further schematic perspective view of the basin shown in Fig. 1, showing the basin from below, wherein the lid is omitted;
- Fig. 3: shows a schematic top view of the basin shown in Figs. 1 and 2, wherein the lid is omitted;
- Fig. 4: shows a schematic perspective view of a liquid conditioning conduit of a basin according to an embodiment;
- Fig. 5: shows a schematic top view of the liquid conditioning conduit shown in Fig. 4;
- Fig. 6: shows a schematic cross-sectional view of the liquid conditioning conduit shown in Figs. 4 and 5, taken along line A-A in Fig. 5;
- Fig. 7: shows a schematic front view of the liquid conditioning conduit shown in Figs. 4-6;
- Fig. 8: shows a further schematic view of the liquid conditioning conduit shown in Figs. 4-7;
- Fig. 9: shows a schematic perspective view of a basin according to an embodiment;
- Fig. 10: shows a schematic perspective view of a basin according to an embodiment;
- Fig. 11: shows a schematic perspective view of a grossing work station with the basin shown in Fig. 10; and
- Fig. 12: shows a flowchart of a method according to an embodiment.

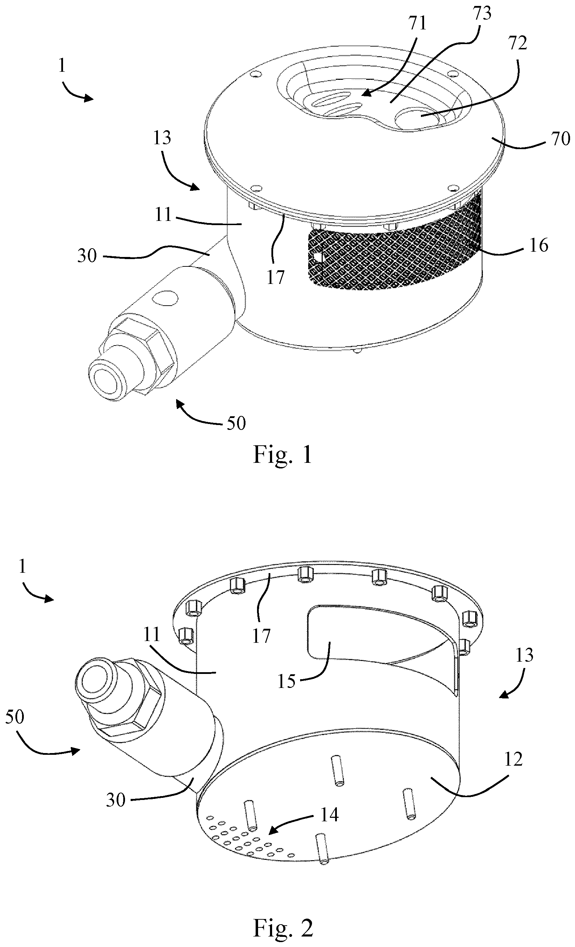

Figs. 1-3 each show a schematic view of a basin 1 according to an embodiment. The basin 1 is for cleaning a tool used during a histology procedure such as a grossing procedure. The tool may be cutlery. The tool may be forceps, a tweezer, a knife and/or a scissor. The tool may be designed to remove a part of a tissue sample or specimen, e.g. to analyse this part in a histological examination. During this, fragments of the tissue sample may be attached to the tool, wherein it is desired to clean the tool and thus remove the fragment in order to avoid a cross contamination.

The basin comprises a cleaning chamber 10 delimited by a side wall 11 and a bottom 12, wherein the side wall 11 extends from the bottom 12. The side wall 11 may comprise a curved cross section, such as a circular or oval cross section. The side wall 11 may form part of a curved (such as cylindrical, conical, and/or only partly curved) body 13 that includes the cleaning chamber 10. In other embodiments, the side wall 11 may also have another cross section, such as a straight cross section or a rectangular cross section.

The cleaning chamber 10 preferably has an internal diameter in a range of 2-50 cm, more preferably of 10-15 cm, and/or a height in a range of 2-40 cm, more preferably of 5-15cm. The cleaning chamber 10, such as the side wall 11 and/or the bottom 12 and/or the body 13, may comprise, or may be made of, metal (such as stainless steel, titanium alloy, aluminum, and/or brass) and/or a plastic material such as polyethylene, polypropylene, PET, PETG, POM, PFA, or the like. A coating or painting may cover at least a part of the side wall 11 and/or the bottom 12 and/or the body 13.

The basin 1 may comprise at least one drain opening 14, 15, preferably in the form of at least one hole. For example, the at least one drain opening 14, 15 comprises a (first) drain opening 14 provided in the bottom 12. The drain opening 14 may comprise a plurality of drain openings 14, which may be arranged according to a matrix or grid. The drain opening 14, 15 may comprise a (second) drain opening 15 provided in the side wall 11. The drain opening 15 may be a single drain opening or may comprise a plurality of drain openings 15. The drain opening 15 may be arranged to function as an overflow opening. As such, the drain opening 15 ensures that liquid within the cleaning chamber 10 remains under a specific level defined by the drain opening 15.

As shown in Fig. 1, the drain opening 15 may comprise a filter 16. By the filter 16, tissue fragments, which are removed from the tool during cleaning of the same within the cleaning chamber 10, can be withheld. The filter 16 may be attached to the side wall 11, preferably in such a way that the filter 16 can be removed therefrom in order to be cleaned and/or replaced. For example, the filter 16 may be removed in order to analyse the tissue fragments accumulated on the side of the filter 16 facing the inside of the cleaning chamber 10.

Additionally, or alternatively, the drain opening 14 may comprise a filter, such as a filter designed in accordance with filter 16. The drain opening 14 may comprise a plurality of drain openings 14 in such a way that the drain openings 14 act as a filter. In other words, each of the drain openings 14 may be sized such that tissue fragments of a specific size cannot flow through the respective drain opening 14 but accumulates on the bottom 12 and in the chamber 10 instead. The plurality of drain openings 14 may be combined with an additional filter, wherein the filter may be arranged within the chamber 10.

In the embodiment shown in Figs. 1-3, the bottom 12 is inclined with respect to the side wall 11, which means that the extending direction of the side wall 11 is not perpendicular to the bottom 12 or a plane surface comprised by the bottom 12. For example, the bottom 12 may be inclined in such a way that liquid arranged on the bottom 12 can flow by gravity towards the drain opening 14 in order to be effectively drained from the cleaning chamber 10. In particular, by the inclination of the bottom 12, the bottom 12 may comprise top and lower portions, wherein the lower portion comprises the drain opening 14.

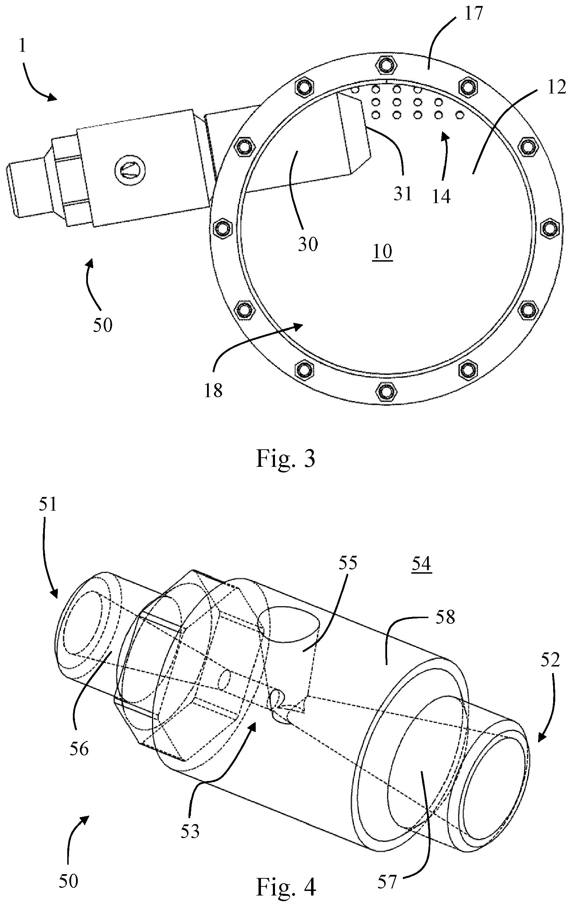

The basin 1 further comprises an inlet conduit 30 arranged to deliver a cleaning liquid via an outlet opening 31 into the cleaning chamber 10. The inlet conduit 30 may comprise, or may be, a pipe or a tube. The cleaning liquid may comprise, or may be, water, such as tap water or mains water. The inlet conduit 30 extends through the side wall 11. In other embodiments, the inlet conduit 30 may extend through the bottom 12, e.g. in addition to extending through the side wall 11. As shown in Fig. 3, a part of the inlet conduit 30 is arranged inside of, and/or protrudes into, the cleaning chamber 10. In other embodiments, the inlet conduit 30 may not be arranged inside of, and/or may not protrude into, the cleaning chamber 10. The outlet opening 31 may then be provided in the side wall 11. If the inlet conduit 30 extends with at least a part into the cleaning chamber 10, the outlet opening 31 may be arranged completely within the cleaning chamber 10. Preferably, the inlet conduit 30 comprises the outlet opening 31.

The inlet conduit 30 is not limited to extend in a specific manner with respect to the side wall 11. For example, the inlet conduit 30 extends not radially with respect to the side wall 11. In other words, the inlet conduit 30 may extend transversely (such as perpendicularly) with respect to a radial direction defined by the side wall 11 and/or the cleaning chamber 10. Preferably, the inlet conduit 30 extends tangentially with respect to the side wall 11.

The inlet conduit 30 may be integrally formed with the side wall 11. In other words, at least a part of the inlet conduit 30 and at least part of the side wall 11 may be part of a monolithic structure. Alternatively, the inlet conduit 30 may be arranged to be detachable from the side wall 11, e.g. by at least a flange-like and/or a threaded connection.

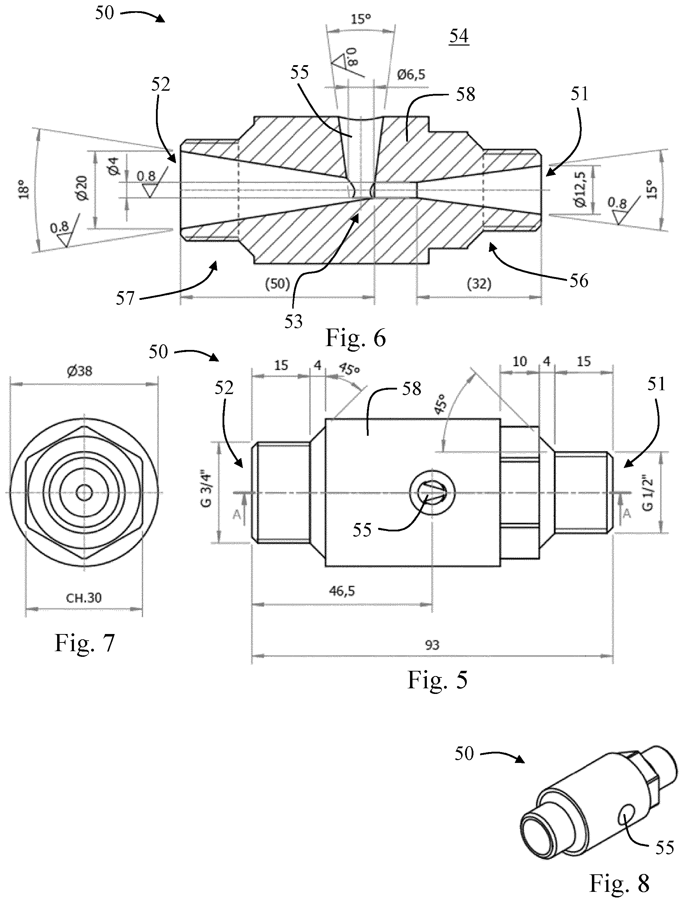

The basin 1 further comprises a liquid conditioning conduit 50, which in the embodiment shown in the figures is provided in the form of a venturi tube. The liquid conditioning conduit 50 is arranged to subject the cleaning liquid to a venturi effect and/or to cavitation upstream of the outlet opening 31. For example, the venturi effect and/or cavitation may be brought about by the liquid conditioning conduit 50 being a venturi tube, which is shown in Figs. 6-8 in more detail.

As is shown in Figs. 5 and 6, the liquid conditioning conduit 50 may comprise an inlet opening 51 connectable to a liquid source such as tap water or mains water. The inlet opening 51 may be connected (directly or indirectly) to the liquid source by a fastening means such as a threaded connection means. The inlet opening 51 may have a diameter in the range of 11-14 mm, such as 12-13 mm. The liquid conditioning conduit 50 may comprise an outlet opening 52 connected to the inlet conduit 30, e.g. by using a fastening means such as a threaded connection means. The outlet opening 52 may have a diameter in a range of 18-22 mm, such as 19-22 mm. Between the inlet opening 51 and the outlet opening 52, the liquid conditioning conduit 50 may comprise a constricted section 53. The constricted section 53 comprises a channel having a diameter smaller than the diameter of the inlet opening 51a and preferably smaller than the diameter of the outlet opening 52. For example, the channel of the constricted section 53 has a diameter of not more than 10mm, e.g. in a range of 1-10 mm, preferably 2-8 mm, more preferably 3-5mm. The channel of the constricted section 53 may comprise at least partly a section with a constant diameter.

Accordingly, a liquid can traverse the liquid conditioning conduit 50 by at least the inlet opening 51, the constricted section 53 (i.e. via the channel of the constricted section 53) and the outlet opening 52 (in this order).

By the liquid flowing from the inlet opening 51 into the constricted section 53, a fluid pressure in the liquid is reduced. Accordingly, the pressure of the liquid in the constricted section 53 is lower than a pressure of the liquid at a position upstream of the constricted section 53, such as in the inlet opening 51. The constricted section 53 may be designed such that the pressure of the liquid within the constricted section 53 falls below a pressure so as to effect a flow of a gas, such as a gas (e.g., air) from an area 54 outside of the liquid conditioning conduit 50, into the constricted section 53 (i.e. in a channel of the constricted section) and thus into the cleaning liquid. Thus, gas (e.g. in the form of bubbles) can be enclosed in the cleaning liquid in at least the constricted section 53 by the venturi effect. The cleaning liquid is conditioned with gas and can then flow downstream through the outlet opening 52, into the inlet conduit 30 and then via the outlet opening 31 into the cleaning chamber 10. The cleaning liquid discharged through the outlet opening 31 is in a particularly agitated and/or turbulent state making the cleaning of a tool within the chamber 10 particularly effective.

To draw the gas from the area 54 (which is provided outside of the liquid conditioning conduit 50) and into the constricted section 53 (where the gas can then be mixed with a liquid), the constricted section 53 may comprise a suction opening 55. In other words, the liquid conditioning conduit 50, may comprise the suction opening 55 to fluidly connect the constricted section 53 with the area 54. Thus, by the liquid in the constricted section 53 having a pressure below the pressure within the area 54, a flow of gas (such as air) can be effected from the area 54 into the constricted section 53 where the gas can then be mixed with the liquid. As shown, the suction opening 55 may be tapered. The suction opening 55 is, however, not limited to a specific form. For example, the suction opening 55 may be cylindrical. With a tapered from, the suction opening may have a taper (or cone) angle in a range of 12-18 degrees, such as 14-16 degrees. The suction opening 55 may have a diameter in the range of 5-7 mm, preferably 6-7 mm.

Flowing into the constricted section 53, the liquid may be also subjected to cavitation. More specifically, flowing into the constricted section 53, the liquid may undergo a pressure drop in such a way that the (static) pressure of the liquid is reduced to below the liquid's vapour pressure. This leads to the formation of small vapour-filled cavities in the liquid. Enclosing the vapour-filled cavities, the liquid can then flow downstream of the constricted section 53 to the outlet opening 52 and into the inlet conduit 30 and then via the outlet opening 31 into the cleaning chamber 10. In the cleaning chamber 10, the cleaning liquid is then in a particularly agitated and/or turbulent state due to the vapour-filled cavities, whereby the cleaning of a tool in the cleaning chamber 10 is particularly effective.

The constricted section 53 may be void of any suction opening designed in accordance with the suction opening 55. In other words, the constricted section 53 may comprise only the channel via which the liquid can flow from the inlet opening 51 to the outlet opening 52. As such, the cleaning liquid within the cleaning chamber 10 may be agitated exclusively by the effect of cavitation in the constricted section 53. In an embodiment, the inlet conduit 30 may be void of any suction opening in addition to the outlet opening 31 and the inlet opening of the inlet conduit 30.

The liquid conditioning conduit 50 may comprise a (upstream) wide section 56 arranged between the inlet opening 51 and the constricted section 53. In other words, the wide section 56 may be arranged downstream of the inlet opening 51 and upstream of the constricted section 53. The wide section 56 comprises a channel that is wider than the channel of the constricted section 53. Accordingly, liquid flowing within the wide section 56 has a pressure that is higher than the pressure of the liquid flowing in the constricted section 53. That is, flowing from the wide section 56 into the constricted section 53, the (cleaning) liquid undergoes a pressure drop that effects the venturi effect and/or cavitation. As shown in Figs. 4 and 6, the channel of the wide section 56 may taper towards the constricted section 53. The channel of the wide section 56 may comprise, or may define, the inlet opening 51. The taper (or cone) angle of the channel of the wide section 56 may be in the range of 13-17 degrees, such as 14-16 degrees.

The liquid conditioning conduit 50 may comprise a (downstream) wide section 57 arranged between the constricted section 53 and the outlet opening 52. In other words, the wide section 57 may be arranged downstream of the constricted section 53 and upstream of the outlet opening 52. The wide section 57 comprises a channel that is wider (e.g. has a larger diameter) than the channel of the constricted section 53. For example, the channel of the wide section 57 may taper towards the constricted section 53. In other words, the channel of the wide section 57 may widen towards the outlet opening 52. The cleaning liquid conditioned at least within the constricted section 53 can flow from there into the wide section 57. The pressure of the so conditioned cleaning liquid builds up as the latter is flowing within the wide section 57 towards the outlet opening 52. However, the wide section 57 may be also omitted, so that, for example, the constricted section 53 directly connects to, or defines, the outlet opening 52. If the wide section 57 is provided, the channel of the wide section 57 may comprise, or may define, the outlet opening 52. The channel of the wide section 57 may have a taper (or cone) angle in the range of 16-20 degrees, such as 17-19 degrees.

The liquid conditioning conduit 50 may comprise a monolithic part 58 that comprises at least the openings 51, 52 and the constricted section 53. Preferably, the monolithic part (or body) may also comprise the fastening means to connect the inlet opening 51 to the liquid source and/or the fastening means to connect the outlet opening 52 to the inlet conduit 30. The monolithic part 58 may comprise the suction opening 55. The monolithic part 58 may comprise the wide section 56 and/or the wide section 57. Preferably, the monolithic part 58 comprises a single channel connecting the openings 51, 52 with one another, wherein the single channel comprises the channel of the constricted section 53 and, preferably, the channel(s) of the wide section 56 and/or wide section 57. The monolithic part 58 may comprise only one channel, namely said single channel, or more than one channel, such as only two channels, namely said single channel and the suction opening 55.

The liquid conditioning conduit 50 may be arranged with respect to the side wall 11 so as to not extend radially with respect to the side wall 11 and/or the cleaning chamber 10. For example, a central axis extending through the openings 51, 52 and the constricted section 53 may extend transversely (such as perpendicularly) with respect to a radial direction defined by the side wall 11. Preferably, this central axis is parallel to a tangent of the side wall 11.

As shown in Figs. 1-3, the liquid conditioning conduit 50 may be arranged as a whole (i.e. completely) outside of the cleaning chamber 10. In this way, a good accessibility of the liquid conditioning conduit 50 can be ensured, e.g. in order to easily remove the liquid conditioning conduit 50 from the inlet conduit 30. In another embodiment, only a part of the liquid conditioning conduit 50 may be arranged outside of the cleaning chamber 10. The respective remaining part of the liquid conditioning conduit 50 may then be arranged within the side wall 11 and/or the cleaning chamber 10.

As shown in Fig. 1, the basin 1 may comprise a lid 70. The lid 70 is arranged to at least partly close the cleaning chamber 10 on a top side thereof. The lid 70 is preferably arranged opposite to the bottom 12. Preferably, the side wall 11 extends between the bottom 12 and the lid 70. In the shown embodiment, in which the bottom 12 is inclined with respect to the side wall 11, the bottom 12 is also inclined with respect to the lid 70. The lid 70 may extend perpendicularly to the side wall 11 and/or may have a round shape. The side wall 11, and/or a flange 17 extending therefrom, may define an opening 18 (Fig. 3), wherein the lid 70 is arranged to cover at least part of the opening 18. The lid 70 may be attached, or attachable, to the side wall 11 and/or the flange 17 by a fastening means such as one or more screws. Preferably, the lid 70 is attached to the flange 17 by using a plurality of fastening means, e.g. evenly distributed along the flange 17. The lid 70 is preferably made of metal or plastic material.

As shown in Fig. 1, the lid 70 may comprise a tool opening 71, 72 arranged to receive a tool being cleaned in the cleaning chamber 10. Accordingly, at least the part of the tool to be cleaned within the cleaning chamber 10 can access the latter via the opening 71, 72. For example, received in the tool opening 71, 72, the tool may be arranged such that the part for handling the tool (which part is usually not provided with tissue fragments) is not arranged in the cleaning chamber 10, wherein the part of the tool that is normally in contact with the tissue specimen is arranged within the cleaning chamber 10. The opening 71, 72 may have a slot-like form and/or a curved form, e.g. a circular and/or oval form. The tool opening 71, 72 may comprise a plurality of tool openings, such as a first tool opening 71 and a second tool opening 72, wherein the tool openings 71, 72 may have different forms. By the different forms, different kinds of tools can be easily received.

The lid 70 may comprise a recess 73, wherein the tool opening 71, 72 extends from a bottom of the recess 73. This achieves the advantage that the tool opening 71, 72 and thus the tool received by the tool opening 71, 72 can be arranged deeper in the cleaning chamber 10, thereby achieving a particularly effective cleaning of the tool by the cleaning liquid within the cleaning chamber 10.

The lid 70 may comprise a mechanical positioning means or structure (such as a protrusion and/or a recess) that can cooperate with a part fixedly arranged with respect to the side wall 11 and/or the outlet opening 31. By this cooperation, the tool opening 71, 72 can be brought in a defined position relative to the side wall 11 and/or outlet opening 31 (and thus with respect to the flowing cleaning liquid within the cleaning chamber 10). Accordingly, it is possible that a tool received by the tool opening 71, 72 can be cleaned in a defined area within the cleaning chamber 10, in which area the flow (turbulent and/or agitated) of the cleaning liquid can clean the tool in a particularly effective manner.

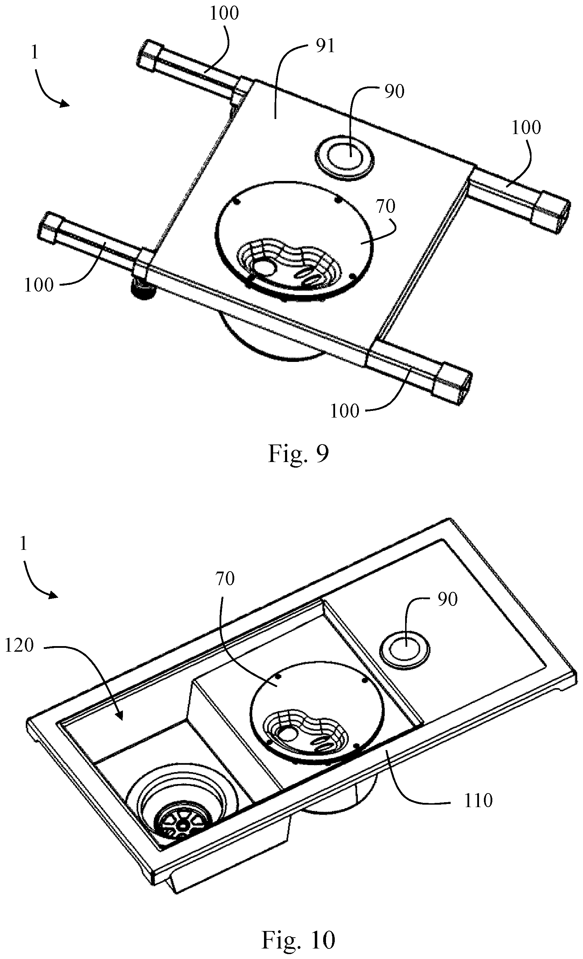

As shown in Figs. 9 and 10, the basin 1 may comprise a user control element 90, such as a touch-sensitive button (e.g. a capacitive and/or resistive element, or a (small) touch screen, or any other control element not requiring mechanical action), for controlling a liquid stream, such as a liquid stream flowing into the liquid conditioning conduit 50. Controlling the liquid stream may comprise at least start of a flow of the liquid stream. For example, the basin 1 may comprise a valve (not shown) arranged upstream of the liquid conditioning conduit 50, e.g. attached to the inlet opening 51. The valve may be a time-controlled valve. The control element 90 may be configured to control the valve. A user may operate the user control element 90, triggering the valve to release a (pressurised) liquid stream (e.g. coming from tap water or mains water) into the liquid conditioning conduit 50. After a defined period of time has been lapsed, the valve may automatically close, thereby stopping the flow of liquid stream into the liquid conditioning conduit 50. As such, an at least partly automatic operation of the basin 1 for cleaning a tool can be provided in an effective manner.

The basin 1 may comprise a plate 91 that receives both the chamber 10 (or the body comprising the chamber 10) and the user control element 90.

The basin 1 may be adapted as an extension and/or add-on module for a (stationary) basin, wherein the latter basin may be part of a laboratory or the same. For example, a water outlet of the (stationary) basin may be connected as the liquid source to the liquid conditioning conduit 50. Used in combination with a (stationary) basin, at least part of the cleaning chamber 10 may extend within the (stationary) basin, i.e. a cleaning chamber of this basin.

The basin 1 is preferably void of any pump. That is, the basin 1 is preferably configured to receive a pressurised liquid from a liquid source not part of the basin 1, such as from tap water or mains water.

As shown in Fig. 9, the basin 1 may comprise a mounting structure 100 (such as one or more arms) arranged to be supported on a rim or side wall of the (stationary) basin so that at least part of the cleaning chamber 10 is arranged within the (stationary) basin, i.e. within the cleaning chamber of this basin. For example, the basin 1 comprises at least one arm 100 (preferably two arms 100) on each lateral side of the basin 1.

As shown in Fig. 10, the basin 1 may comprise a supporting structure 110 receiving a (sub-) basin 120 such as a basin for washing hands. The supporting structure 110 may be a chassis. The supporting structure 110 may also receive the user control element 90. The cleaning chamber 10, or the body comprising the chamber 10, is received by the supporting structure 110.

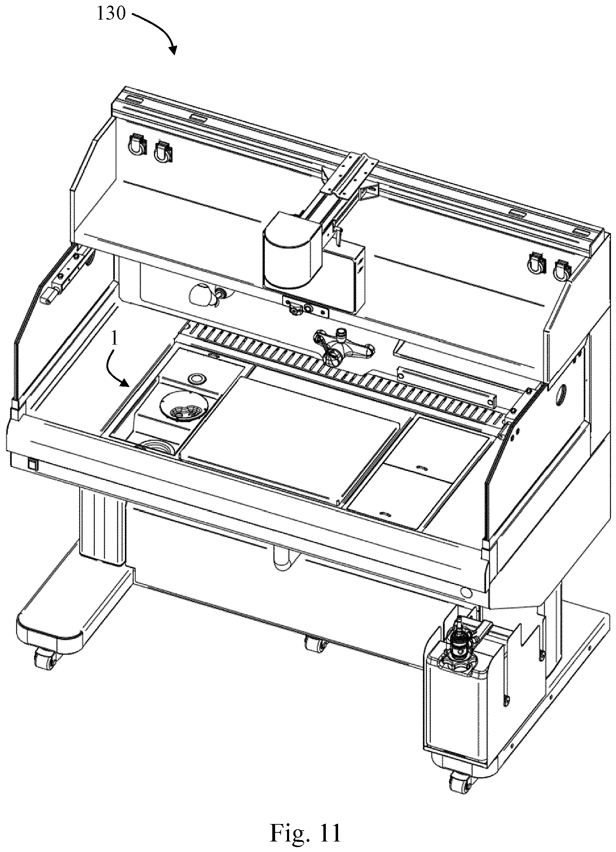

The basin 1 may be part of a system or station provided, or being used during a procedure, in a histological pathway, in particular during or after grossing. In particular, a tissue sample station may comprise the basin 1. As shown in Fig. 11, the tissue sample station may be a grossing station 130.

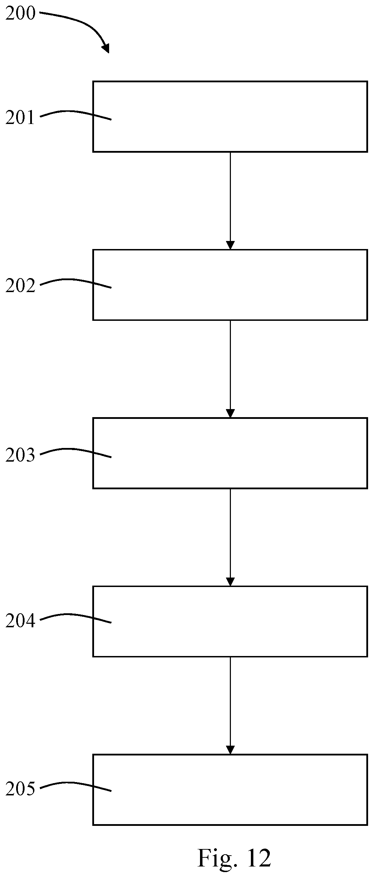

With respect to Fig. 12, an exemplary method for cleaning a tool used during a histological procedure such as grossing procedure is described. The method 200 comprises the following steps:
In step 201, the basin 1 is provided. For example, the basin 1 is provided in a (stationary) water basin (e.g. as an add-on module) or in a tissue sample station (work station) such as a grossing workstation of a pathology/histology laboratory. For a cleaning liquid flowing in the basin 1, the basin 1 is preferably connected to a liquid supply system (such as tap water or mains water) and/or to a supply system of another cleaning liquid. The cleaning liquid can include a liquid (such as water) and/or or a liquid cleaning solution (such as, but not limited to, enzymatic cleaner, hydrogen peroxide, sodium hypochlorite, ionic or anionic based cleaner or the like).
In step 202, the cleaning liquid is subjected, by the liquid conditioning conduit 50, to a venturi effect and/or to cavitation upstream of the outlet opening 31. Prior to this, the tool to be cleaned may be inserted and received in the proper tool opening 71, 72 (e.g. a slot) of the lid 70, wherein then the timed valve is activated by operating the user control element 90 (e.g. by passing the hand close to the automatic detection sensor) and/or by acting on a manual lever/pushbutton. The liquid, such as water, through the valve is sent into the inlet opening 51 of the liquid conditioning conduit 50. Due to the venturi effect in the conduit 50, the liquid that flows inside from the inlet opening 51 (first inlet) generates a reduction of pressure in the constricted section 53 comprising the suction opening 55 (second inlet; normally open at atmospheric pressure), causing the air or any other gas to be sucked inside via the suction opening 55 and mixed with the liquid in the constricted section 53. The liquid in the constricted section 53 is mixed with the gas flows out of the suction opening 55.

In another embodiment, a pressure of the liquid in the constricted section 53 is obtained (e.g. by the constricted section 53 and by adjusting the liquid's flow speed in relation to the temperature of the liquid), which pressure is lower than the vapor pressure of the liquid flowing through the constricted section 53, whereby a quick phase transition from liquid to vapor occurs (cavitation), thus generating vapor bubbles in the liquid by the cavitating liquid conditioning conduit 53, e.g. a venturi tube. (For The generation of cavitation through a venturi tube see, e.g.: Hongbo Shi, Mingda Li, Petr Nikrityuk, Qingxia Liu; Experimental and numerical study of cavitation flows in venturi tubes: From CFD to an empirical model; Chemical Engineering Science, Volume 207, 2019, Pages 672-687.)

In both cases of step 202 (gas mixed into the liquid through the venturi effect tube or bubbles generated in the liquid through the cavitation induced by the liquid conditioning conduit 50), the result is a liquid conditioned such that there is turbulence, or agitation, in the liquid.

In step 203, which is after step 202, the cleaning liquid conditioned in step 202 is delivered, by the inlet conduit 30 and the outlet opening 31, into the cleaning chamber 10. The cleaning chamber 10 receives the turbulent liquid from the liquid conditioning conduit 50. The liquid may start filling the cleaning chamber 10. The drain openings 14 with an optional filter at the bottom 12 of the cleaning chamber 10 for draining the liquid may be provide such that liquid is drained via the drain openings 41 at a draining flow rate lower than the incoming flow rate of the flow through the outlet opening 21. As such, the incoming liquid is more than the drained liquid, whereby the cleaning chamber 10 starts filling. After a certain amount of time, the liquid level may reach the drain opening 15 in the upper part of the sidewall 11 of the cleaning chamber 10, draining the liquid exceeding this level. The drain opening 15 may have a dimension that allows a drain flow higher than the incoming flow, thus maintaining the liquid at a specific level to permit common tools to be submerged at the right position in relation to the liquid.

In step 204, which may be after or during step 203, the tool used during the histology procedure (such as grossing) is cleaned by the cleaning liquid delivered by step 203. The delivered cleaning liquid is still in the conditioned state obtained in step 202, whereby the tool can be cleaned in a particularly effective manner. The turbulent liquid arriving, via the inlet conduit 30 and the outlet opening 31, in the cleaning chamber 10 from the liquid conditioning conduit 50 mechanically acts on the surface of the one or more tool(s) placed inside the cleaning chamber 10, detaching the tissue fragments from the tool(s). The one or more tool openings 71, 72 on the top of the lid 70 help to place the tools in a defined area within the cleaning chamber 10, which area is particularly effective for cleaning, such as right in front of the connection of the liquid conditioning conduit 50 (with the inlet conduit 30), i.e. in front of the injection of the liquid flow, where the turbulence is at the maximum. The one or more tool openings 71, 72 may be positioned to permit common tools to be accommodated at the right position in relation to the liquid flow in the cleaning chamber 10.

The optional tangent position of the liquid conditioning conduit 50 in relation to the cleaning chamber 10 facilitates the creation of a vortex inside the cleaning chamber 10 that adds a further mechanical force for detaching the tissue fragments from the tools. Moreover, the lid 70 has the function to avoid that some drops of the liquid sprayed out by the turbulence may reach the operator.

In an optional step 205, which is after step 204, the cleaned tool is reused in a histology procedure such as a grossing procedure. For example, after a preset amount of time (e.g., 10 seconds), the valve closes automatically, thus stopping the liquid flow. At this time, the incoming flow is null and then the cleaning chamber 10 empties progressively, draining the remaining liquid through the drain openings 14 of the bottom 12. In an embodiment, in which the bottom 12 of the body 13 is oblique, the slope of the bottom 12 conveys the liquid to the drain openings 14, ensuring a complete emptying of the cleaning chamber 10. Thereby, it can be avoided that the dirty liquid used for cleaning the tool remains inside the cleaning chamber 10. For example during emptying the cleaning chamber 10, the operator can remove the cleaned tool(s) from the basin 1 and reuse it for the grossing, or any other histology procedure, of the following specimen without cross-contamination.

The filter(s) 16 provided in one or more of the drain openings 14, 15 can hold the tissue fragments removed from the tools inside the cleaning chamber 10. This can be useful to avoid an unnoticed or unintentional loss of useful tissue specimen that may have been wrongly left on the tools before the cleaning. Both filters may be removed to be cleaned and avoid drain clogging.

In case of fresh specimen or if a molecular biology analysis is required for the tissue specimens, a decontamination of the tools may be required after the cleaning, before being reused, such as between steps 204 and 205. This can be achieved by rinsing the cleaned tool in a proper decontaminant, such as an enzymatic detergent, hydrogen peroxide, sodium hypochlorite, etc.

The method 200 may comprise one, more or all of the following optional steps: before step 202, using the tool for performing a histology procedure (such as grossing) with respect to a tissue specimen; before step 202, inserting the tool in the tool opening 71, 72 of the lid 70; activate the valve, e.g. by operating the user control element 90, and start the cleaning of the tool within the cleaning chamber 10; cleaning the tool for a specific (cleaning) time; when the specific (cleaning) time has lapsed, automatically deactivating the valve and thus stopping the cleaning of the tool within the cleaning chamber 10; removing the cleaned tool from the basin 1; between steps 203 and 204 or after step 204, such as between steps 204 and 205, additional cleaning (such as decontaminating) the tool, such as by using a different cleaning agent, such as a decontaminant and/or a cleaning agent comprising a detergent.

## Claims

1. A basin (1) for cleaning a tool used during a histology procedure such as a grossing procedure, the basin (1) comprising:
- a cleaning chamber (10) delimited by a side wall (11) extending from a bottom (12);
- an inlet conduit (30) arranged to deliver a cleaning liquid via an outlet opening (31) into the cleaning chamber (10), wherein the inlet conduit (30) extends through the side wall (11) and/or the bottom (12); and
- a liquid conditioning conduit (50) arranged to subject the cleaning liquid to a venturi effect and/or to cavitation upstream of the outlet opening (31).

2. The basin (1) according to claim 1, wherein the liquid conditioning conduit (50) comprises, or is, a venturi tube.

3. The basin (1) according to claim 1 or 2, wherein the liquid conditioning conduit (50) comprises: an inlet opening (51) connectable to a liquid source, an outlet opening (52) connected to the inlet conduit (30), and a constricted section (53) arranged between the inlet opening (51) and the outlet opening (52) of the liquid conditioning conduit (50).

4. The basin (1) according to claim 3, wherein the constricted section (53) comprises a suction opening (55) through which a gas, such as air, can be drawn from an area (54) outside of the liquid conditioning conduit (50) and into a liquid within the constricted section (53).

5. The basin (1) according to claim 3, wherein the constricted section (53), or the liquid conditioning conduit (50), is void of any suction opening through which a gas, such as air, can be drawn from an area outside of the liquid conditioning conduit and into a liquid within the constricted section.

6. The basin (1) according to any one of the preceding claims, wherein the side wall (11) has a curved cross-section, and wherein the inlet conduit (30) and/or the liquid conditioning conduit (50) do/does not extend radially with respect to the side wall (11) and/or cleaning chamber (10), or extend/s tangentially with respect to the side wall (11).

7. The basin (1) according to any one of the preceding claims, further comprising a lid (70), wherein the lid (70) comprises a tool opening (71, 72) arranged to receive a tool being cleaned in the cleaning chamber (10), wherein the lid (70) is preferably arranged to cover at least part of an opening (18) defined by the side wall (11) or by a flange (17) extending from the side wall (11).

8. The basin (1) according to any one of the preceding claims, further comprising a drain opening (14, 15) for draining liquid from the cleaning chamber (10), wherein, preferably, the bottom (12) and/or the side wall (11) comprises the drain opening (14, 15).

9. The basin (1) according to claim 8, wherein the drain opening (14, 15) comprises a filter (16).

10. The basin (1) according to any one of the preceding claims, wherein at least part of the bottom (13) is inclined, preferably such that liquid on the bottom (12) can flow by gravity towards the drain opening (14)..

11. The basin (1) according to any one of the preceding claims, wherein at least part of the liquid conditioning conduit (50) is arranged outside of the cleaning chamber (10), and/or wherein at least part of the inlet conduit (30) is arranged inside of the cleaning chamber (10).

12. The basin (1) according to any one of the preceding claims, further comprising:
- a valve arranged upstream of the liquid conditioning conduit (50), wherein the valve is preferably at least a time-controlled valve; and/or
- a pressure and/or flow regulator arranged to control a pressure and/or flow of a liquid flowing towards, or in, the inlet conduit (30); and/or
- a heater arranged to heat, preferably upstream of the cleaning chamber (10), the cleaning liquid or an ingredient of the cleaning liquid.

13. The basin (1) according to any one of the preceding claims, further comprising a user control element (90) for controlling a liquid stream, such as for controlling the valve and/or the pressure and/or flow regulator and/or the heater, wherein, preferably, a plate (91) receives the cleaning chamber (10) and the user control element (90).

14. A method (200) for cleaning a tool used during a histology procedure such as a grossing procedure, the method comprising the steps of:
a) providing (201) a basin (1) according to any one of the preceding claims,
b) subjecting (202) a cleaning liquid, by the liquid conditioning conduit (50), to a venturi effect and/or cavitation upstream of the outlet opening (31),
c) delivering (203), by the inlet conduit (30) and the outlet opening (31), the conditioned cleaning liquid into the cleaning chamber (10),
d) cleaning (204), by the delivered cleaning liquid, the tool in the cleaning chamber (10), and
e) optionally reusing (205) the cleaned tool in a histology procedure such as a grossing procedure.

15. Use of the basin (1) according to any one of claims 1-13 in a histological pathway, such as during or after grossing.
